# EUROPÄISCHE PATENTSCHRIFT

(11) **EP 1 622 549 B1**
(45) Veröffentlichungstag und Bekanntmachung des Hinweises auf die Patenterteilung: **12.12.2007**
(21) Anmeldenummer: 03709543.7
(22) Anmeldetag: 11.04.2003
(51) Int. Cl.: A61F 2/44

(54) **VERANKERUNGSMITTEL FÜR ZWISCHENWIRBELIMPLANTATE**
ANCHORING MEANS FOR INTERVERTEBRAL IMPLANTS
MOYENS D'ANCRAGE POUR IMPLANTS INTERVERTEBRAUX

(43) Veröffentlichungstag der Anmeldung: 08.02.2006
(73) Patentinhaber: Synthes GmbH, 4436 Oberdorf (CH)
(72) Erfinder: BAUMGARTNER, Daniel, CH-4702 Oensingen (CH); MATHIEU, Claude, CH-8002 Zürich (CH); BURRI, Adrian, CH-3900 Brig (CH)
(74) Vertreter: Lusuardi, Werther
(86) Internationale Anmeldenummer: PCT/CH2003/000240
(87) Internationale Veröffentlichungsnummer: WO 2004/089256

(56) Entgegenhaltungen:
- WO-A-99/32055
- DE-A- 19 509 317
- US-A1- 2002 138 142
- US-B1- 6 176 881

## Beschreibung

Die Erfindung bezieht sich auf Verankerungsmittel für Zwischenwirbelimplantate, gemäss dem Oberbegriff des Patentanspruchs 1 sowie auf ein Zwischenwirbelimplantat mit zwei Verankerungsteilen gemäss dem Oberbegriff des Patentanspruchs 9 und auf ein Verfahren zur Befestigung eines Zwischenwirbelimplantates an angrenzenden Wirbelkörpern gemäss dem Oberbegriff des Patentanspruchs 16.

Zwischenwirbelimplantate, welche beispielsweise als Bandscheibenendoprothesen ausgestaltet sein können und nach dem Entfernen einer beschädigten, natürlichen Bandscheibe oder eines beschädigten Nukleus einer Bandscheibe in den Zwischenwirbelraum zwischen zwei benachbarten Wirbelkörpern eingeführt werden, müssen an den Endflächen der benachbarten Wirbelkörper fixiert werden, damit sich das Implantat mit der Zeit nicht verschieben kann. Bei der Befestigung des Implantates an den Endplatten der Wirbelkörper wird zwischen primärer und sekundärer Stabilisation unterschieden. Die primäre Stabilisation ist direkt nach der Operation notwendig und wird vorzugsweise durch Einbringen von am Implantat angebrachten Verankerungsmitteln in die Endplatten an den benachbarten Wirbelkörpern erzeugt. Die sekundäre Stabilisation wird durch das Anwachsen des Knochens am Implantat erreicht, wobei jedoch mit einer Dauer von ca. 6 Wochen bis zu einer ausreichenden Fixierung des Implantates zu rechnen ist.

Aus der US 5,683,465 SHINN ist eine Bandscheibenendoprothese bekannt, welche in einer Ausführungsform mittels durch die aussenstehend am Implantat angebrachten Deckplatten durchführbaren Stiften an den Endplatten der angrenzenden Wirbelkörper fixiert wird. Nachteilig an dieser Fixation mittels dieser Stifte ist, dass die Stifte entweder vor der Einführung der Bandscheibenendoprothese in den Zwischenwirbelraum an den Deckplatten befestigt werden müssen, was beim Einführen des Implantates in den Zwischenwirbelraum eine vergrösserte Distraktion der beiden Wirbelkörper erfordert, oder dass die Stifte andernfalls nach dem Einführen des Implantates in den Zwischenwirbelraum einzeln in die Endplatten der angrenzenden Wirbelkörper eingepresst werden müssen, was eine erhöhte Operationsdauer zur Folge haben kann.

US 2002/0138142 beschreibt ein Zwischen wirbel implantat gemäß dem Oberbegriff von Anspruch 1. Die Verankerungsmittel werden hierbei stirnseitig auf den Implantatkörper aufgesetzt. Dies erfordert ebenfalls eine vergrösserte Distraktion der Wirbelkörper beim Einsetzen des Implatats.

Hier will die Erfindung Abhilfe schaffen. Der Erfindung liegt die Aufgabe zugrunde, Verankerungsmittel für Zwischenwirbelimplantate zu schaffen, welche zum Einführen des Implantates in den ausgeräumten Zwischenwirbelraum in eine erste Position bringbar sind, wo sie endständig nicht über die Deckplatten hinausragen, und auf einfache Weise nach dem Einführen des Implantates in eine zweite, arretierbare Position bringbar sind, wo die Verankerungsmittel in die Endplatten der angrenzenden Wirbelkörper eingepresst sind und zur primären Stabilisation des Implantates dienen.

Die Erfindung löst die gestellte Aufgabe mit Zwischenwirbelimplantaten, welche die Merkmale des Anspruchs 1 aufweisen.

Das erfindungsgemässe Zwischenwirbelimplantat umfasst zwei axial endständige Abschlussplatten, deren aussenstehende Oberflächen zur Anlage an die Deckplatte, respektive die Grundplatte der zwei angrenzenden Wirbelkörper dienen, und zwei Verankerungsmittel.

Die Verankerungsmittel dienen zur Fixierung eines Zwischenwirbelimplantates an den Endplatten von Wirbelkörpern und umfassen im wesentlichen ein Verankerungsteil mit einer Zentralachse, einem das Verankerungsteil in Richtung der Zentralachse durchdringenden Hohlraum und zwei quer zur Zentralachse stehende Stirnflächen, mindestens zwei über eine der Stirnflächen hinausragende Dorne, welche in die Endplatte eines Wirbelkörpers einpressbar sind, und Befestigungsmittel, mittels welcher das Verankerungsteil zusammen mit den Dornen an einem Zwischenwirbelimplantat lösbar arretierbar ist.

Die Abschlussplatten können durch die Hohlräume in den Verankerungsteilen durchgeführt werden, so dass die Verankerungsteile axial relativ zu den Abschlussplatten verschiebbar sind. Dadurch sind die Vorteile erreichbar, dass
- vor dem Einführen des Zwischenwirbelimplantates in den Zwischenwirbelraum die Verankerungsteile axial verschoben werden können, bis die Dorne nicht über die endständigen Stirnflächen der Abschlussplatten hinausragen und somit beim Einführen des Zwischenwirbelimplantates in den Zwischenwirbelraum die angrenzenden Wirbelkörper nur minimal auseinander gespreizt werden müssen; und
- nach dem Einführen des Zwischenwirbelimplantates in den Zwischenwirbelraum die beiden Verankerungsteile mit einem einfachen Instrument verschiebbar sind, bis die Dorne in die Grund-, respektive Deckplatte der angrenzenden Wirbelkörper eingepresst sind.

Die Befestigungsmittel können beispielsweise quer zur Zentralachse des Verankerungsteiles an dem Zwischenwirbelimplantat einrastbar und elastisch deformierbar sein, quer zur Zentralachse in das Verankerungsteil einschieb- oder einschraubbar sein, oder durch eine Konusverbindung zwischen Hohlraumwand und Zwischenwirbelimplantat ausgeführt sein.

In einer bevorzugten Ausführungsform sind die Befestigungsmittel quer zur Zentralachse des Verankerungsteils elastisch deformierbar und ragen im nicht-deformierten Zustand in den Hohlraum im Verankerungsteil. Elastisch deformierbare Befestigungsmittel weisen den Vorteil auf, dass die Verankerungsteil einstückig herstellbar sein können und die Gefahr, ein Bestandteil zu verlieren, vermieden werden kann.

Vorzugsweise sind diese Befestigungsmittel als Haken mit gegen die Zentralachse gerichteten Nasen ausgestaltet.

In einer anderen Ausführungsform sind die Befestigungsmittel im Hohlraum des Verankerungsteiles angeordnet. Dadurch ist der Vorteil erreichbar, dass das Verankerungsteil ohne axial über die Stirnflächen hinausragende Teile herstellbar ist und beispielsweise das Einpressen der Dorne in die Grund-, respektive Deckplatte eines angrenzenden Wirbelkörpers mittels eines geeigneten chirurgischen Instrumentes nicht durch vorstehende Teile behindert wird.

In wiederum einer anderen Ausführungsform sind die Haken in Vertiefungen in der zur Zentralachse parallelen Hohlraumwand derart eingelassen, dass bei nicht quer zur Zentralachse deformierten Haken die Nasen der Haken in den Hohlraum hineinragen und bei quer zur Zentralachse deformierten Haken die Haken inklusive ihrer gegen die Zentralachse gerichteten Nasen in der Vertiefung aufnehmbar sind, so dass das Zwischenwirbelimplantat in den Hohlraum einführbar ist.

In einer weiteren Ausführungsform ist das Verankerungsteil ringförmig ausgestaltet, wobei die zur Zentralachse orthogonale Querschnittsfläche des Hohlraumes und/oder die durch die äussere Mantelfläche eingegrenzte und zur Zentralachse orthogonale Querschnittsfläche der Verankerungsteiles Kreisflächen, elliptische Flächen, ovale Flächen oder polygonale Flächen sein können.

In einer weiteren Ausführungsform sind die Abschlussplatten spielfrei in den Hohlräumen der Verankerungsteile gelagert und parallel zur Zentralachse relativ zu den Abschlussplatten verschiebbar. Dadurch ist der Vorteil erreichbar, dass das Zwischenwirbelimplantat nach Fixierung der Verankerungsmittel in der Grundrespektive der Deckplatte der angrenzenden Wirbelkörper kein radiales Spiel aufweist.

In einer anderen Ausführungsform umfassen die Abschlussplatten zweite Befestigungsmittel, in welche die Befestigungsmittel an den Verankerungsteilen zu Eingriff bringbar sind. Diese zweiten Befestigungsmittel können beispielsweise darin bestehen, dass die Abschlussplatten des Zwischenwirbelimplantates an ihren Mantelfächen zur Zentralachse parallele Kerben, welche zur Aufnahme der an den Haken angebrachten Nasen dienen. Die Ausgestaltung mit Kerben hat den Vorteil, dass durch die in die Kerben eingerasteten Nasen die Abschlussplatten gegen Verdrehung relativ zu den Verankerungsteilen gesichert werden können.

In einer weiteren Ausführungsform weisen die Befestigungsmittel an den Verankerungsteilen gegenüber am Zwischenwirbelimplantat angebrachten zweiten Befestigungsmitteln Spiel auf, derart, dass bei fixierten Befestigungsmitteln geringe Verdrehungen der Verankerungsteile um die Zentralachse relativ zu den Abschlussplatten zugelassen werden. Dadurch ist der Vorteil erreichbar, dass Torsionsbewegungen der angrenzenden Wirbelkörper, welche innerhalb eines gewissen Bereiches zulässig sind, durch die Verbindung zwischen den Verankerungsteilen und dem Zwischenwirbelimplantat zugelassen werden.

In wiederum einer anderen Ausführungsform bestehen die zweiten Befestigungsmittel darin, dass die Abschlussplatten mit axial aussenstehenden, im Durchmesser verjüngten Segmenten ausgestaltet sind, so dass die Nasen der Haken einrasten können.

Ein mögliches Verfahren zur Befestigung eines Implantates, insbesondere eines Zwischenwirbelimplantates an den Endplatten der beiden angrenzenden Wirbelkörper umfasst im wesentlichen die folgenden Schritte
a) Ermöglichen eines Zuganges zum Zwischenwirbelraum, mittels eines anterolateralen, ventralen lateralen, transperitonialen oder retroperitonialen chirurgischen Eingriffes;
b) Distrahieren der beiden an den Zwischenwirbelraum angrenzenden Wirbelkörper;
c) Ausräumen des Zwischenwirbelraumes;
d) Einführen des Zwischenwirbelimplantates mit gegeneinander zusammengeschobenen Verankerungsmitteln. Dabei werden die beiden Verankerungsteile soweit gegeneinander zusammengeschoben bis die Dorne nicht mehr über die aussenstehenden Oberflächen der Abschlussplatten hinausragen;
e) Verschieben der Verankerungsteile axial auseinander, bis die Dorne ausreichend in die Grund-, respektive die Deckplatte der angrenzenden Wirbelkörper eingepresst sind; und
f) Fixieren der Befestigungsmittel am Zwischenwirbelimplantat. Falls die Befestigungsmittel elastisch ausgeführt sind, erfolgt das Fixieren derselben selbsttätig ohne weiteres Zutun des Operateurs sobald die Verankerungsteile bis zu ihrer axialen Endposition auseinander geschoben sind. Wenn die Befestigungsmittel jedoch als Schrauben oder ähnliche Mittel ausgestaltet sind, müssen diese mit einem geeigneten Instrument fixiert werden.

Die Erfindung und Weiterbildungen der Erfindung werden im folgenden anhand der teilweise schematischen Darstellungen mehrerer Ausführungsbeispiele noch näher erläutert.

Es zeigen:
Fig. 1 einen Schnitt durch eine Ausführungsform der erfindungsgemässen Verankerungsmittel;
Fig. 2 eine Aufsicht auf die in Fig. 1 dargestellte Ausführungsform der erfindungsgemässen Verankerungsmittel;
Fig. 3 einen Schnitt durch zwei an einem Zwischenwirbelimplantat angeordneten Verankerungsmittel gemäss der in den Fig. 1 und 2 dargestellten Ausführungsform;
Fig. 4 einen Ausschnitt aus einer Wirbelsäule mit einem implantierten Zwischenwirbelimplantat und zwei Verankerungsmitteln gemäss der in den Fig. 1 und 2 dargestellten Ausführungsform; und
Fig. 5 einen Längsschnitt durch ein Zwischenwirbelimplantat mit zwei Verankerungsmitteln gemäss in den Fig. 1 und 2 dargestellten Ausführungsform.

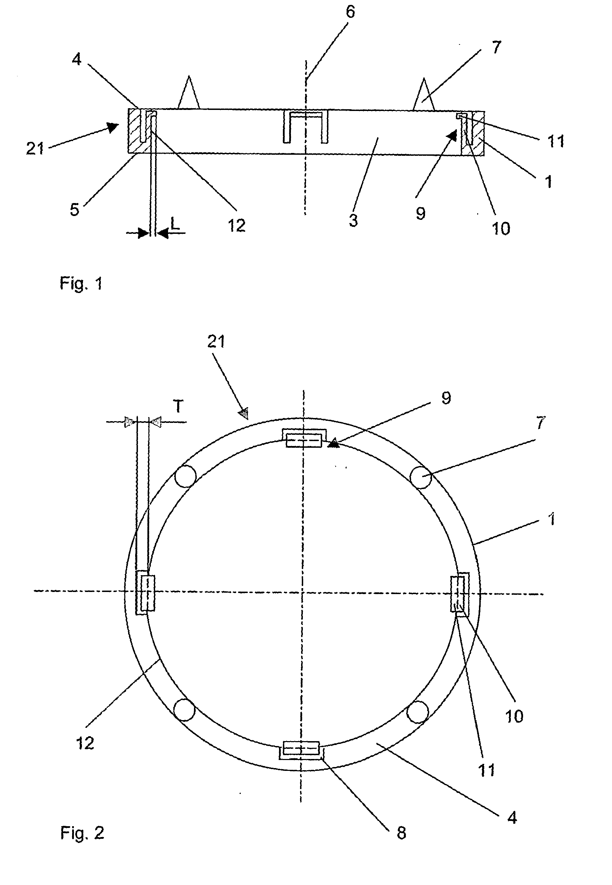

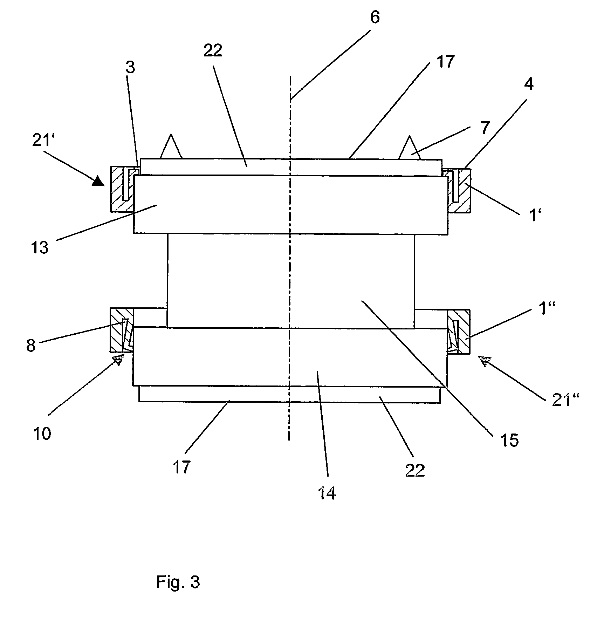

In den Fig. 1 und 2 ist eine bevorzugte Ausführungsform der erfindungsgemässen Verankerungsmittel 21 dargestellt, welche im wesentlichen ein Verankerungsteil 1 mit einer Zentralachse 6 und einem das Verankerungsteil 1 parallel zur Zentralachse 6 durchdringenden Hohlraum 3, parallel zur Zentralachse 6 mehrere, beispielsweise vier Dorne 7 und Befestigungsmittel 9 umfassen. Das Verankerungsteil 1 ist hier in einem zur Zentralachse 6 orthogonalen Querschnitt betrachtet kreisringförmig ausgestaltet, kann aber auch innen und/oder aussen ellipsenförmig, oval, nierenförmig oder polygonförmig ausgestaltet sein, und weist eine erste Stirnfläche 4 und parallel dazu eine zweite Stirnfläche 5 auf. Die beiden Stirnflächen 4;5 sind zur Zentralachse 6 orthogonal angeordnet. Die vier Dorne 7 sind mit dem Verankerungsteil 1 einstückig und stehen senkrecht auf der ersten Stirnfläche 4. Die Dorne 7 können beispielsweise derart ausgestaltet sein, dass sie sich wie hier dargestellt axial gegen ihr freies Ende hin verjüngen oder sie können endständig an ihren freien Enden spitzig oder konvex ausgebildet sein, so dass sie bei der Implantation durch Verschieben des Verankerungsteiles 1 parallel zur Zentralachse 6 in die Endplatte eines angrenzenden Wirbelkörpers einpressbar sind. Als Befestigungsmittel 9 sind an der zur Zentralachse 6 parallelen Hohlraumwand 12 gleichmässig auf dem Umfang verteilt vier quer zur Zentralachse 6 elastisch deformierbare Haken 10 angeordnet, deren Nasen 11 bei der ersten Stirnfläche 4 des Verankerungsteiles 3 angeordnet sind und in den Hohlraum 3 ragen. Die Haken 10 sind mit den Nasen 11 an einem in den Hohlraum 3 eingeführten Zwischenwirbelimplantat 15 (Fig. 3) lösbar einrastbar. Die Haken 10 sind in Vertiefungen 8 in der Hohlraumwand 12 so angeordnet, dass bei nicht deformierten Haken 10 lediglich die Nasen 11 quer zur Zentralachse 6 in den Hohlraum 3 ragen. Die Vertiefungen 8 weisen senkrecht zur Zentralachse 6 gemessen ein Tiefe T auf, während die Nasen 11 ebenfalls senkrecht zur Zentralachse 6 gemessen eine Länge L aufweisen, wobei L < T ist.

Fig. 3 zeigt zwei gleiche Verankerungsmittel 21';21 entsprechend der in den Fig. 1 und 2 beschriebenen Ausführungsform, welche je an einem Ende eines Zwischenwirbelimplantates 15 angeordnet sind, wobei die an den Verankerungsteilen 1';1" angebrachten Dorne 7 endständig über die Stirnflächen 17 des Zwischenwirbelimplantat 15 hinausragen. Die Stirnflächen 17 des Zwischenwirbelimplantates 15 können wie hier dargestellt planar, oder aber auch gewölbt ausgebildet sein. Dabei ist das eine Verankerungsmittel 21' in seiner zweiten, arretierten Position dargestellt während sich das andere Verankerungsmittel 21" in einer ersten Position befindet, in welcher es parallel zur Zentralachse 6 so weit über das Zwischenwirbelimplantat 15 geschoben ist, dass die Dorne 7 nicht über die endständige Stirnfläche 17 des Zwischenwirbelimplantates 15 ragen. Beim Einführen eines Zwischenwirbelimplantates 15 in den Hohlraum 3 können sich die Haken 10 in die Vertiefungen 8 ausbiegen, so dass das Zwischenwirbelimplantat 15 parallel zur Zentralachse 6 durch den Hohlraum 3 und an den Nasen 11 vorbei schiebbar ist. Dies ist beispielhaft am Verankerungsteil 1" dargestellt. Die Abschlussplatten 13; 14 des Zwischenwirbelimplantates 15 weisen axial endständig im Durchmesser verjüngte Segmente 22 auf, so dass die Nasen 11 der Haken 10 in den Absatz, welcher an den Abschlussplatten 13;14 durch die verjüngten Segmente 22 gebildet wird, einrasten können. Dadurch ist erreichbar, dass die aussenstehenden Stirnflächen 17 der Abschlussplatten 13;14 an der Grundplatte, respektive der Deckplatte der angrenzenden Wirbelkörper anliegen. Da die Stirnfläche 4 des Verankerungsteiles 1 somit nicht an den angrenzenden Wirbelkörpern anliegt, ist gewährleistet, dass lediglich das Zwischenwirbelimplantat 15 axial lasttragend ist und die Last auf den gesamten Stirnflächen 17 übertragen wird.

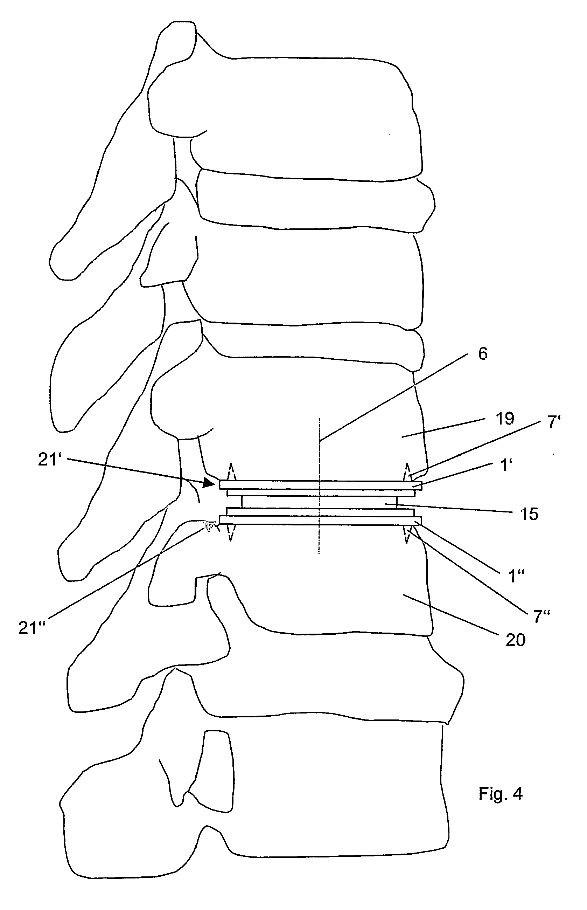

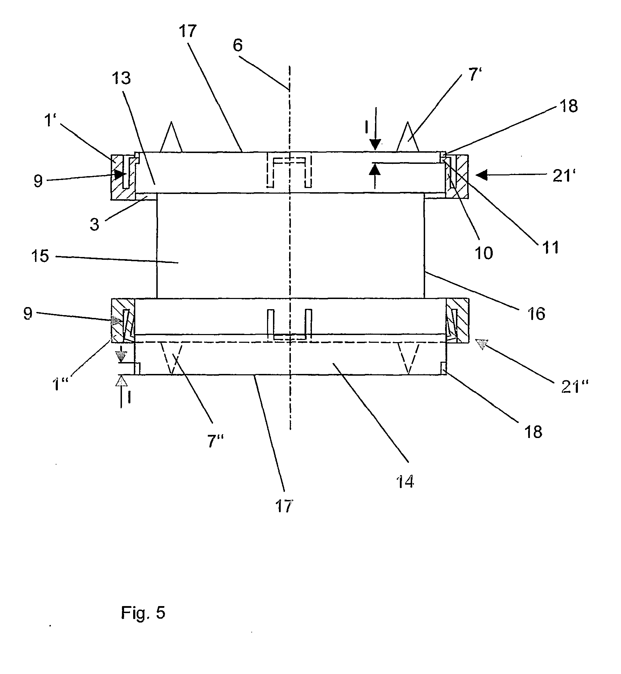

Fig. 4 zeigt einen Ausschnitt aus einer Wirbelsäule zusammen mit einem zwischen zwei benachbarten Wirbelkörpern 19;20 eingeführten Zwischenwirbelimplantat 15. Das Zwischenwirbelimplantat 15 ist je mit einem Verankerungsmittel 21';21" an der Endplatte des angrenzenden Wirbelkörpers 19;20 fixiert. Zur Fixation der Verankerungsmittel 21';21" an den Wirbelkörpern 19;20 sind die an den Verankerungsteilen 1';1" angebrachten Dorne 7';7" in die Endplatten der Wirbelkörper 19;20 eingepresst. Bei der Implantation des Zwischenwirbelimplantates 15 in den ausgeräumten Zwischenwirbelraum werden die Verankerungsteile 1';1" soweit über das Zwischenwirbelimplantat 15 geschoben, dass die Dorne 7 nicht über die endständige Stirnfläche 17 des Zwischenwirbelimplantates 15 vorstehen (Fig. 3). Erst nach dem Einschieben des Zwischenwirbelimplantates 15 zusammen mit zwei Verankerungsteilen 1';1" in den ausgeräumten Zwischenwirbelraum werden das untere und der obere Verankerungsteil 1";1' mittels einer Spreizzange gegen die an das Zwischenwirbelimplantat 15 angrenzenden Wirbelkörper 19;20 verschoben und die Dorne 7 in die Endplatten der angrenzenden Wirbelkörper 19;20 gepresst. Nachdem die Dorne 7 vollständig in die Endplatten eingepresst wurden und die Verankerungsteile 1';1" ihre Endposition erreicht haben rasten die beiden Haken 10 (Fig. 2) mit ihren Nasen 11 beispielsweise an den endständigen Stirnflächen 17 des Zwischenwirbelimplantates, oder in zu den Nasen 11 komplementären Vertiefungen 18 (Fig. 5) an der zur Zentralachse 6 parallelen äusseren Mantelfläche 16 des Zwischenwirbelimplantates 15 ein.

In Fig. 5 ist eine Ausführungsform eines Zwischenwirbelimplantates 15 mit je einem axial endständig angeordneten Verankerungsmittel 21';21" dargestellt. Die Verankerungsmittel 21';21" entsprechen den in den Fig. 1 und 2 beschriebenen Verankerungsmitteln und umfassen je ein Verankerungsteil 1';1" und an der axial endständigen Stirnfläche 4 der Verankerungsteile 1';1" Dorne 7';7". Das Zwischenwirbelimplantat 15 ist mit je einer axial endständigen Abschlussplatte 13;14 ausgestattet, wobei im zur Zentralachse 6 orthogonalen Querschnitt betrachtet die Abschlussplatten 13;14 komplementär zu den Hohlräumen 3 der Verankerungsteile 1';1" ausgestaltet sind. Die Abschlussplatten 13;14 sind an der äusseren Mantelfläche 16 mit Kerben 18 versehen, welche analog wie die Befestigungsmittel 9 an den Verankerungsteilen 1';1" auf dem Umfang verteilt sind und zu den Nasen 11 an den Befestigungsmitteln 9 komplementär ausgestaltet sind. Ferner weisen die Kerben 18 parallel zur Zentralachse 6 gemessen eine Länge I auf und münden in die axial endständigen Stirnflächen 17 der zum Zwischenwirbelimplantat 15 zählenden Abschlussplatten 13;14. Die Länge I ist derart bemessen, dass bei einer axialen Verschiebung der Verankerungsteil 21';21" gegen die Stirnflächen 17 des Zwischenwirbelimplantates 15 die Nasen 11 der Haken 10 in die Kerben 18 einrasten. Die aussenliegenden Stirnflächen 17 des Zwischenwirbelimplantates 15 stehen axial über die endständigen Stirnflächen 4 der Verankerungsteile 1';1" vor, so dass gewährleistet ist, dass die Last von den beiden angrenzenden Wirbelkörpern über die Stirnflächen 17 auf das Zwischenwirbelimplantat 15 übertragen wird. Das obere Verankerungsmittel 21' ist hier mit eingerasteten Befestigungsmitteln 9 dargestellt, während das untere Verankerungsmittel 21" auf der Abschlussplatte 14 so weit gegen die entgegengesetzt angeordnete Abschlussplatte 14 verschoben ist, dass die Dorne 7" nicht über die Stirnfläche 17 des Zwischenwirbelimplantates 15 ragen. Analog zu Fig. 3 sind die Befestigungsmittel 9 des unteren Verankerungsmittels 21" quer zur Zentralachse 6 deformiert und in die Vertiefungen 8 im Hohlraum 3 des Verankerungsteiles 1" gepresst.

## Patentansprüche

1. Zwischenwirbelimplantat (15) mit zwei Verankerungsmitteln (21) zur Fixierung des Zwischenwirbelimplantates (15) an die Endplatte eines Wirbelkörpers (19;20), wobei
A) jedes Verankerungsmittel (21) ein Verankerungsteil (1) umfasst, welches eine Zentralachse (6) und zwei quer zur Zentralachse (6) stehende Stirnflächen (4;5) umfasst; und
B) jedes Verankerungsmittel (21) mindestens zwei über eine der Stirnflächen (4;5) vorstehende, zur Zentralachse (6) parallele und in eine Endplatte eines Wirbelkörpers (19;20) einpressbare Dome (7) umfasst,
C) das Verankerungsteil (1) einen parallel zur Zentralachse (6) durchgehenden Hohlraum (3) umfasst; und
D) das Verankerungsteil (1) Befestigungsmittel (9) umfasst, mittels welcher das Verankerungsteil an einem Zwischenwirbelimplantat (15) lösbar arretierbar ist ;
**dadurch gekennzeichnet, dass**
E) das Zwischenwirbelimplantat (15) endständig je eine die Zentralachse (6) schneidende Abschlussplatte (13;14) umfasst; und
F) dass die Abschlussplatten (13;14) durch die Hohlräume (3) in den Verankerungsteilen (1) durchführbar sind.

2. Zwischenwirbelimplantat (15) nach Anspruch 1, **dadurch gekennzeichnet, dass** die Befestigungsmittel (9) quer zur Zentralachse (6) elastisch deformierbar sind und im nicht-deformierten Zustand in den Hohlraum (3) ragen.

3. Zwischenwirbelimplantat (15) nach Anspruch 2, **dadurch gekennzeichnet, dass** die Befestigungsmittel (9) quer zur Zentralachse (6) elastisch deformierbare Haken (10) mit gegen die Zentralachse (6) gerichteten Nasen (11) sind.

4. Zwischenwirbelimplantat (15) nach Anspruch 3, **dadurch gekennzeichnet, dass** die Haken (10) im Hohlraum (3) angeordnet sind.

5. Zwischenwirbelimplantat (15) nach einem der Ansprüche 1 bis 4, **dadurch gekennzeichnet, dass** die Befestigungsmittel (9) und das Verankerungsteil (1) einstückig sind.

6. Zwischenwirbelimplantat (15) nach einem der Ansprüche 3 bis 5, **dadurch gekennzeichnet, dass** der Hohlraum (3) Vertiefungen (8) umfasst, worin die Haken (10) angeordnet sind.

7. Zwischenwirbelimplantat (15) nach Anspruch 6, **dadurch gekennzeichnet, dass** die Vertiefungen (8) senkrecht zur Zentralachse (6) eine Tiefe T aufweisen und die Nasen (11) senkrecht zur Zentralachse (6) gemessen eine maximale Länge L aufweisen, wobei L < T ist.

8. Zwischenwirbelimplantat (15) nach einem der Ansprüche 1 bis 7, **dadurch gekennzeichnet, dass** das Verankerungsteil (1) ringförmig ausgestaltet ist und die zur Zentralachse (6) orthogonale Querschnittsfläche des Hohlraumes (3) und/oder die durch die äussere Mantelfläche eingegrenzte zur Zentralachse (6) orthogonale Querschnittsfläche des Verankerungsteiles (1) Kreisflächen, elliptische Flächen, polygonale Flächen oder ovale Flächen sind.

9. Zwischenwirbelimplantat (15) nach einem der Ansprüche 1 bis 8, **dadurch gekennzeichnet, dass** die Form der Hohlräume (3) an die Abschlussplatten (13;14) angepasst ist.

10. Zwischenwirbelimplantat (15) nach Anspruch 9, **dadurch gekennzeichnet, dass** die Abschlussplatten (13;14) spielfrei in den Hohlräumen (3) der Verankerungsteile (1) gelagert und parallel zur Zentralachse (6) verschiebbar sind.

11. Zwischenwirbelimplantat (15) nach einem der Ansprüche 1 bis 8, **dadurch gekennzeichnet, dass** die Verankerungsteile (1) bei an den Abschlussplatten (13;14) fixierten Befestigungsmitteln (9) bezüglich Verdrehung um die Zentralachse (6) Spiel aufweisen.

12. Zwischenwirbelimplantat (15) nach einem der Ansprüche 1 bis 11, **dadurch gekennzeichnet, dass** die Abschlussplatten (13;14) zweite Befestigungsmittel umfasst, in welche die Befestigungsmittel (9) an den Verankerungsteile (1) in Eingriff bringbar sind.

13. Zwischenwirbelimplantat (15) nach Anspruch 12, **dadurch gekennzeichnet, dass** es eine äussere Mantelfläche (16) aufweist und als zweite Befestigungsmittel quer zur Zentralachse (6) in die Mantelfläche (16) eindringende Kerben (18) zur teilweisen Aufnahme der Befestigungsmittel (9) umfasst.

14. Zwischenwirbelimplantat (15) nach einem der Ansprüche 1 bis 13, **dadurch gekennzeichnet, dass** die Abschlussplatten (13;14) axial aussenstehende, im Durchmesser verjüngte Segmente (22) umfassen.

## Claims

1. An intervertebral implant (15) with two anchoring means (21) to fix the intervertebral implant (15) on the end plate of a body (19, 20) of the vertebra wherein
A) each anchoring means (21) comprises an anchoring part (1) comprising a central axis (6) and two end faces (4, 5) transverse to the central axis (6),
B) each anchoring means (21) comprises at least two spikes (7) that protrude past the end faces (4, 5), are parallel to the central axis (6) and can be pressed into an end plate of a body (19, 20) of the vertebra,
C) the anchoring part (1) comprises a hollow space (3) passing through parallel to the central axis (6), and
D) the anchoring means (1) comprises fastening means (9) by means of which the anchoring means can be detachably locked on an intervertebral implant (15),
**characterised in that**
E) the intervertebral implant (15) comprises a closing plate each (13, 14) that intersects the central axis (6), and
F) the closing plates (13, 14) can pass through the hollow spaces (3) in the anchoring parts (1).

2. An intervertebral implant (15) according to claim 1, **characterised in that** the fastening means (9) can be elastically deformed transversely to the central axis (6) and in the non-deformed state protrude into the hollow space (3).

3. An intervertebral implant (15) according to claim 2, **characterised in that** the fastening means (9) are hooks (10) that can be elastically deformed transversely to the central axis (6) with lugs (11) facing the central axis (6).

4. An intervertebral implant (15) according to claim 3, **characterised in that** the hooks (10) are provided in the hollow space (3).

5. An intervertebral implant (15) according to any one of claims 1 to 4, **characterised in that** the fastening means (9) and the anchoring part (1) are integral.

6. An intervertebral implant (15) according to any one of claims 3 to 5, **characterised in that** the hollow space (3) comprises depressions (8), wherein the hooks (10) are provided.

7. An intervertebral implant (15) according to claim 6, **characterised in that** perpendicularly to the central axis (6) the recesses (8) have a depth T and the maximum length of the lugs (11), measured perpendicularly to the central axis (6), is *L*, while *L<T.*

8. An intervertebral implant (15) according to any one of claims 1 to 7, **characterised in that** the anchoring part (1) has an annular construction and the cross-sectional surface of the hollow space (3) at right angles to the central axis (6) and/or the cross-sectional surface of the anchoring part (1) bordered by the external sheathing surface at right angles to the central axis (6) are circular surfaces, elliptical surfaces, polygonal surfaces or oval surfaces.

9. An intervertebral implant (15) according to any one of claims 1 to 8, **characterised in that** the shape of the hollow space (3) is made to suit the closing plates (13, 14).

10. An intervertebral implant (15) according to claim 9, **characterised in that** the closing plates (13, 14) are mounted without clearance in the hollow spaces (3) of the anchoring parts (1) and can be displaced relative to the central axis (6).

11. An intervertebral implant (15) according to any one of claims 1 to 8, **characterised in that** the anchoring parts (1) have a clearance for rotation about the central axis (6) with fastening means (9) fixed on the closing plates (13, 14).

12. An intervertebral implant (15) according to any one of claims 1 to 11, **characterised in that** the closing plates (13, 14) comprise second fastening means, in which the fastening means (9) can be engaged on the anchoring parts (1).

13. An intervertebral implant (15) according to claim 12, **characterised in that** it has an external sheathing surface (16) and as second fastening means it comprises depressions (18) protruding into the sheathing surface (16) transversely to the central axis (6) for the partial accommodation of the fastening means (9).

14. An intervertebral implant (15) according to any one of claims 1 to 13, **characterised in that** the closing plates (13, 14) have axially projecting segments (22) with reduced diameters.

## Revendications

1. Implant intervertébral (15) comprenant deux moyens d'ancrage (21) pour fixer l'implant intervertébral (15) sur le plateau d'extrémité d'un corps vertébral (19 ; 20), dans lequel
A) chaque moyen d'ancrage (21) comprend une partie d'ancrage (1) qui a un axe central (6) et deux faces frontales (4 ; 5) disposées transversalement à l'axe central (6) ; et
B) chaque moyen d'ancrage (21) comprend au moins deux broches (7) dépassant de l'une des faces frontales (4 ; 5), qui sont parallèles à l'axe central (6) et qui peuvent être enfoncées dans un plateau d'extrémité d'un corps vertébral (19 ; 20),
C) la partie d'ancrage (1) comprend un espace creux (3) ininterrompu parallèle à l'axe central (6) ; et
D) la partie d'ancrage (1) comprend des moyens de fixation (9) à l'aide desquels la partie d'ancrage peut être bloquée de manière amovible sur un implant intervertébral (15) ;
**caractérisé en ce que**
E) l'implant intervertébral (15) comprend à chaque extrémité une plaque terminale (13 ; 14) coupant l'axe central (6) ; et
F) les plaques terminales (13 ; 14) peuvent être déplacées à travers les espaces creux (3) dans les parties d'ancrage (1).

2. Implant intervertébral (15) selon la revendication 1, **caractérisé en ce que** les moyens de fixation (9) sont déformables de manière élastique transversalement à l'axe central (6) et rentrent dans l'espace creux (3) à l'état non déformé.

3. Implant intervertébral (15) selon la revendication 2, **caractérisé en ce que** les moyens de fixation (9) sont des crochets (10), déformables de manière élastique transversalement à l'axe central (6), présentant des ergots (11) orientés vers l'axe central (6).

4. Implant intervertébral (15) selon la revendication 3, **caractérisé en ce que** les crochets (10) se trouvent dans l'espace creux (3).

5. Implant intervertébral (15) selon l'une quelconque des revendications 1 à 4, **caractérisé en ce que** les moyens de fixation (9) et la partie d'ancrage (1) sont formés d'une seule pièce.

6. Implant intervertébral (15) selon l'une quelconque des revendications 3 à 5, **caractérisé en ce que** l'espace creux (3) comprend des évidements (8) dans lesquels se trouvent les crochets (10).

7. Implant intervertébral (15) selon la revendication 6, **caractérisé en ce que** les évidements (8) présentent une profondeur T perpendiculairement à l'axe central (6) et les ergots (11) présentent une longueur maximale L mesurée perpendiculairement à l'axe central (6), où L < T.

8. Implant intervertébral (15) selon l'une quelconque des revendications 1 à 7, **caractérisé en ce que** la partie d'ancrage (1) a une forme annulaire, et la surface de section transversale de l'espace creux (3) orthogonale à l'axe central (6) et/ou la surface de section transversale de la partie d'ancrage (1) orthogonale à l'axe central (6), délimitée par l'enveloppe externe, sont des surfaces circulaires, des surfaces elliptiques, des surfaces polygonales ou des surfaces ovales.

9. Implant intervertébral (15) selon l'une quelconque des revendications 1 à 8, **caractérisé en ce que** la forme des espaces creux (3) est adaptée aux plaques terminales (13 ; 14).

10. Implant intervertébral (15) selon la revendication 9, **caractérisé en ce que** les plaques terminales (13 ; 14) sont logées sans jeu dans les espaces creux (3) des parties d'ancrage (1) et peuvent être déplacées parallèlement à l'axe central (6).

11. Implant intervertébral (15) selon l'une quelconque des revendications 1 à 8, **caractérisé en ce que** les parties d'ancrage (1) présentent un certain jeu au niveau des moyens de fixation (9) fixés sur les plaques terminales (13 ; 14) pour la rotation autour de l'axe central (6).

12. Implant intervertébral (15) selon l'une quelconque des revendications 1 à 11, **caractérisé en ce que** les plaques terminales (13 ; 14) comprennent des deuxièmes moyens de fixation avec lesquels les moyens de fixation (9) peuvent être mis en prise au niveau des parties d'ancrage (1).

13. Implant intervertébral (15) selon la revendication 12, **caractérisé en ce qu'**il présente une enveloppe externe (16) et qu'il comprend, en tant que deuxièmes moyens de fixation, des encoches (18), pénétrant transversalement à l'axe central (6) dans l'enveloppe (16), destinées à loger partiellement les moyens de fixation (9).

14. Implant intervertébral (15) selon l'une quelconque des revendications 1 à 13, **caractérisé en ce que** les plaques terminales (13 ; 14) comprennent des segments (22) dont le diamètre est rétréci, situés à l'extérieur sur le plan axial.
